# EUROPEAN PATENT SPECIFICATION

(11) **EP 1 656 147 B1**
(45) Date of publication and mention of the grant of the patent: **25.07.2018**
(21) Application number: 04764262.4
(22) Date of filing: 18.08.2004
(51) Int. Cl.: A61K 31/192, A61K 31/603

(54) **DIFLUNISAL FOR THE TREATMENT OF CANCER**
VERWENDUNG VON DIFLUNISAL ZUR BEHANDLUNG VON KREBS
UTILISATION DU DIFLUNISAL POUR LE TRAITEMENT DU CANCER

(30) Priority: 19.08.2003 US 495896 P; 19.08.2003 DE 10338090
(43) Date of publication of application: 17.05.2006
(73) Proprietor: Kreutz, Werner, 79219 Staufen (DE)
(72) Inventor: Kreutz, Werner, 79219 Staufen (DE)
(74) Representative: Best, Michael
(86) International application number: PCT/EP2004/009276
(87) International publication number: WO 2005/016354

(56) References cited:
- WO-A-96/28144
- WO-A-98/58639
- WO-A-02/085327
- WO-A-02/085342
- WO-A-02/098403
- US-A- 5 679 638
- TEICHER B A ET AL: "CYCLOOXYGENASE AND LIPOXYGENASE INHIBITORS AS MODULATORS OF CANCER THERAPIES" CANCER CHEMOTHERAPY AND PHARMACOLOGY, SPRINGER VERLAG, BERLIN, DE, vol. 33, no. 6, 1994, pages 515-522, XP000676574 ISSN: 0344-5704
- COLLET J-P ET AL: "Colorectal cancer prevention by non-steroidal anti-inflammatory drugs: Effects of dosage and timing" BRITISH JOURNAL OF CANCER 1999 UNITED KINGDOM, vol. 81, no. 1, 1999, pages 62-68, XP002302007 ISSN: 0007-0920
- MUELLER MARKUS ET AL: "Interstitial methotrexate kinetics in primary breast cancer lesions", CANCER RESEARCH, vol. 58, no. 14, 15 July 1998 (1998-07-15) , pages 2982-2985, ISSN: 0008-5472
- HWANG D H ET AL: "NATIONAL CANCER INSTITUTE WORKSHOP ON CHEMOPREVENTIVE PROPERTIES OF NONSTEROIDAL ANTI-INFLAMMATORY DRUGS: ROLE OF COX-DEPENDENT AND -INDEPENDENT MECHANISMS", NEOPLASIA, DOYMA, BARCELONA, ES, vol. 4, no. 2, 1 March 2002 (2002-03-01), pages 91-97, XP001096301, ISSN: 0212-9787, DOI: 10.1038/SJ.NEO.7900226
- Nils Fogh-Andersen ET AL: "Composition of Interstitial Fluid", General Clinical Chemistry, 1 January 1995 (1995-01-01), pages 1522-1525, XP055065290, Retrieved from the Internet: URL:http://www.clinchem.org/content/41/10/ 1522.full.pdf [retrieved on 2013-06-04]
- Martin Ellmerer ET AL: "Measurement of interstitial albumin in human skeletal muscle and adipose tissue by open-flow microperfusion", Am J Physiol Endocrinol Metab 278, 1 January 2000 (2000-01-01), pages E352-E356, XP055065291, Retrieved from the Internet: URL:http://ajpendo.physiology.org/content/ 278/2/E352.full.pdf#page=1&view=FitH [retrieved on 2013-06-04]

## Description

The present invention relates to the compound Diflunisal for use in the treatment of cancer. Surprisingly, Diflunisal is active even if it is administered not in form of a synergistic composition with another active ingredient but administered alone, in particular, if Diflunisal is administered in a certain dosage.

Medicaments according to the prior art which are employed in chemotherapy as a rule only achieve partial success, i.e. they do not lead to a final cure. Moreover, anti-tumor agents employed in the prior art frequently act only on a certain tumor category. A further disadvantage of many known chemotherapeutics is their often harmful side effects as chemotherapeutics generally have a cytostatic effect on proliferating tissue. Many of the known chemotherapeutics are also unsatisfactory in the control of metastases formation, and this is one of the main reasons why until now cancer still is a disease which is difficult to treat and in many cases leads to the death of the patient.

The fact that tumor tissue in an extracellular medium has a lowered average pH of about 6.5 to 7.0 (and the pH can even fall to 5 on the cancer cell surface) while the pH in the normal tissue and in the blood is approximately 7.2 to 7.5 is known and described e.g. in DE-A 44 07 484 and in Tumor Biol., 1994, 15:304-310. Each type of tumor has an intrinsic average intercellular pH which e.g. in the case of breast tumors is about 6.7 and in the case of colon tumors is about 6.9.

In the above mentioned publications it is disclosed that due to the lowering of the pH range in tumor cells the natural immune defense is blocked, as the body's own defense cells react to cancer target cells with full cytotoxicity only in a slightly basic medium of a pH of more than 7. DE-A 44 07 484 therefore proposes to bring the acidic external medium of cancer cells to the normal physiological pH level of 7 to 7.5 and thereby to control the cancer cells by means of the body's own immune defense. For this purpose the acidic external medium of cancer cells is brought to a physiological pH of 7 to 7.5 either by artificial basification measures or by the prevention of the acidification process itself.

The medicaments described in DE-A 44 07 484 represents an advance in cancer therapy, but it would be desirable to have available medicaments which besides the body's own immune defense selectively control tumor cells and thus can be used as relatively low side effect chemotherapeutics.

WO 96/30003 proposes to use those compounds for the control of tumor tissue which at a pH of less than 7 are protonated or release a substance the protonated compound or the released substance having a more strongly destructive effect on cells than the unprotonated compound or the compound before release of the substance. WO 96/30003 also generally discloses mixtures of two or more compounds. These compounds do already have a good anti-tumor effect, but there still is a need for medicaments which have an excellent anti-tumor effect, in particular at pH values of 7.0 or below, in particular in the range of 6.5 to 7.0.

WO 98/58639 discloses synergistic compositions for the selective control of tumor tissue. The synergistic compositions comprise at least two different benzoic derivatives which are selected from a list which comprises the compound diflu = 5-(2,4-difluorophenyl)salicylic acid which is also known as "Diflunisal". According to WO 98/58639 Diflunisal is only active when administered together with another compound to form a synergistic mixture.

Despite the high anti-tumor activity of the synergistic compositions disclosed in WO 98/58639, there is still a need for further medicaments which have excellent anti-tumor effects, in particular at pH values of 7.0 or below and more particularly in the range of 6.5 to 7.0.

Diflunisal is a known NSAID and as such was used to reduce proliferation in the case of colon and colorectal cancer (Life Science 70, 2001, 37-88 and British Journal of Cancer, 1999, 81 (1), 62-68) and for the treatment and prevention of cellular abnormalities of the female reproductive tract (US-A1-2003/0004143). According to the British Journal of Cancer, 1999, 81 (1), 62-68 the maximum recommended daily dose in cases of colon cancer and rectal cancer was 1000 mg/day, and according to Life Science 70, 2001, 37-48 the active concentration of Diflunisal is suprapharmacological and intrinsically cytotoxic. Diflunisal was not believed to have apoptotic capability.

Unexpectedly, it was found that Diflunisal has an excellent anti-cancer and anti-tumor activity, even without the co-administration of a further active ingredient such as a benzoic acid derivative as disclosed in WO 98/58639 even for treating cancers other than colon cancer, rectal cancer and cellular abnormalities of the reproductive tract of a female mammal. Unexpectedly and contrary to a widespread prejudice in the prior art, Diflunisal is not only active in reducing proliferation in certain specific cancers but has a significant apoptotic efficiency if administered in a certain well-defined dose or, even better, according to a suitable dosage regimen. Unexpectedly, Diflunisal can be used as sole active ingredient in cancer therapy, and it is not necessary to use it as part of a combination therapy with other active ingredients such as other benzoic acid derivatives as disclosed e.g. in WO 98/58639.

While Diflunisal is sufficiently active on its own without co-administration of other pharmaceutically active ingredients, it is, of course, also possible to combine the Diflunisal with another active ingredient, when Diflunisal is administered in a manner that a certain interstitial concentration is achieved, and therefore the present invention provides Diflunisal for use in the treatment of cancer, wherein the Diflunisal-containing medicament is adapted for administration so that a molecular ratio of 4:1 to 6:1 interstitial Diflunisal:interstitial albumin is provided, wherein the interstitial albumin concentration is measured in the lymph fluid system.

Diflunisal (5-[2,4-difluorophenyl]-salicylic acid) exhibits two extraordinary qualities which are of fundamental interest for cancer therapy:
Firstly, it induces pH-dependent apoptosis in lactate-transporting cells and secondly it causes pH-dependent "pore formation" in cancer cells. "Pore formation" means that cancer cells become "leaky" leading to concentration gradients collapse, which in consequence causes cell death.

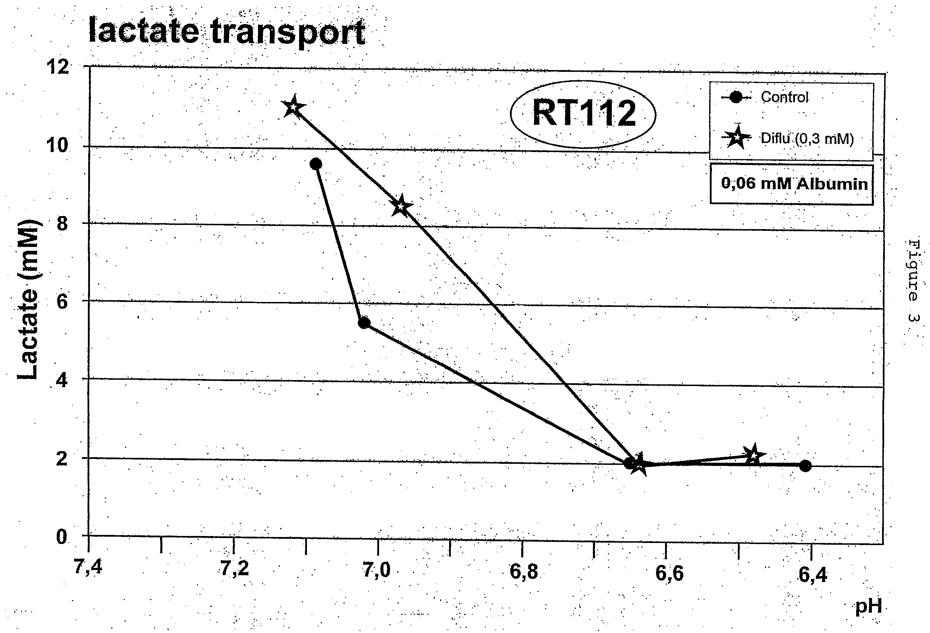

It was found that the pH region in which apoptosis is induced by Diflunisal depends on the Diflunisal concentration applied. It was also found that Diflunisal influx into the cancer cells is mediated by albumin and is coupled to lactate efflux, which means that Diflunisal influx is coupled as an antiport to lactate efflux. This Diflunisal antiport is pH- and concentration-dependent and is ruled by the state of albumin loading with Diflunisal. The Diflunisal antiport can be measured as an accelerated lactate efflux as shown in the examples (figure 3). Thus, the Diflunisal concentration needed to induce apoptosis is dependent on the concentration of albumin present in the interstitial milieu. In the interstitial milieu normally an average albumin concentration of 0.17 mM is established. This value follows from measurements of the albumin concentration in the lymph fluid system.

If Diflunisal is orally administered, in most cases the resorption does not occur ad libidum but only up to an amount which leads to a precise measurable concentration in blood of 250 µg/ml, i.e. the resorption of Diflunisal shows saturation behavior. 250 µg/ml corresponds to a 1.00 mM Diflunisal solution. This saturation concentration is reached at 50 mg/kg Diflunisal application. Even if 100 mg/kg Diflunisal is administered, 1.00 mM will not be exceeded. Since Diflunisal represents a small molecule, it is to be expected that by resorption Diflunisal distributes equimolarly within the fluid systems of the organs and blood because of the lack of diffusion barriers, i.e. also the interstitial milieu should adopt a 1.00 mM Diflunisal concentration.

It appears that the saturation behavior of Diflunisal is ruled by the binding capacity of albumin for Diflunisal. If at an albumin concentration of 0.17 mM saturation occurs at 1 mM Diflunisal concentration, each albumin should be able to bind 1.0 mM/0.17 mM ≈ 6 molecules Diflunisal.

In vitro measurements revealed that at maximum 12 molecules of Diflunisal can be bound by one molecule of albumin. At lower Diflunisal to albumin ratios there seem to exist binding preferences for 3 and 6 Diflunisal per albumin.

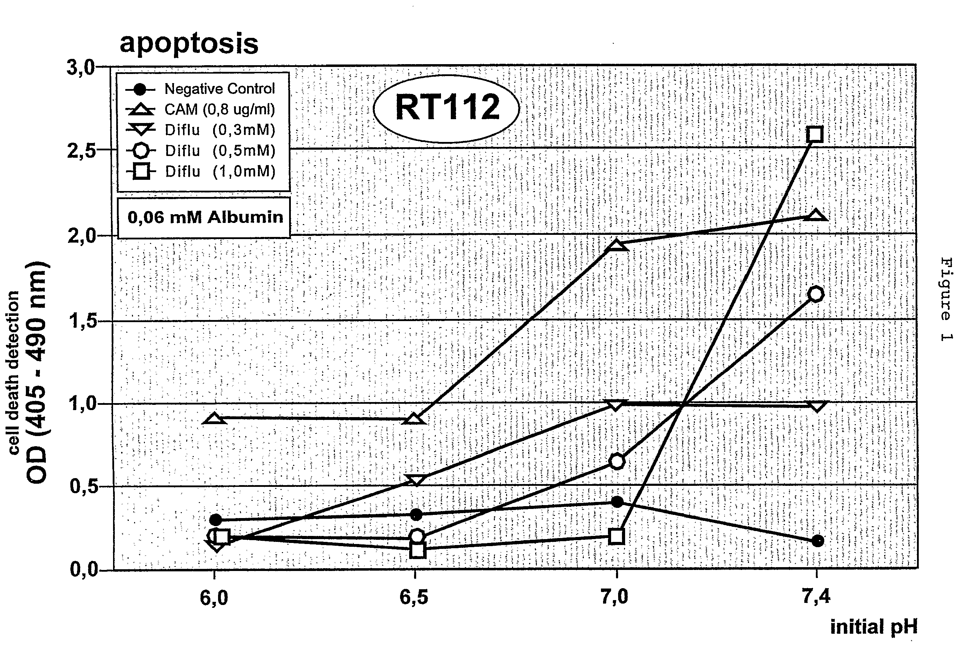

Under in vivo condition of 0.17 mM albumin, if only 3 Diflunisal molecules are bound to albumin, only pore formation at pH < 6.5 happens and also lactate antiport at pH 6.6-7.4. With 4 to 6 bound Diflunisal to albumin apoptosis at pH 6.0-7.2 occurs. If the ratio of Diflunisal/albumin is elevated to 7-9, apoptosis is achieved at pH 7.0-7.4 with an exact onset point at pH 7.0 (figure 1).

At 4:1 to 6:1 Diflunisal/albumin ratio in vivo corresponds to 0.7-1.0 mM interstitial Diflunisal concentration. A 7:1 to 9:1 ratio means a concentration of 1.2-1.5 mM interstitial Diflunisal. For therapy purpose apoptosis action at pH 7.0-7.4 is not acceptable. Within this pH range also normal tissues, which may also perform lactate transport to a lower level, could be killed by Diflunisal application. To avoid this intolerable side effect, it must be guaranteed that with Diflunisal therapies Diflunisal must only act via four to six Diflunisal/albumin ratio.

This is guaranteed when Diflunisal application is performed solely orally. It is an outstanding and remarkable feature of Diflunisal that when exclusively administered orally no overdosage can happen because of its resorption saturation behavior at 1.0 mM as outlined above. Therefore, therapy in principle even with a single high oral depot dose could be performed which, however, is not tolerable for most patients..

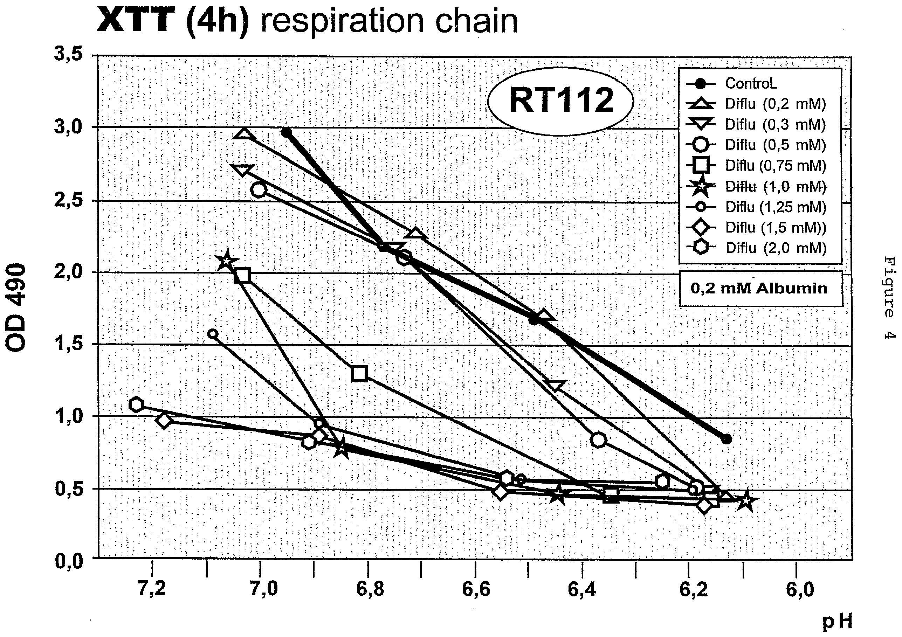

Furthermore, the activity of the respiration chain of cancer cells is representative for the effectivity of the therapy (figure 4). The activity of the mitochondrial respiration chain is a sensitive indication for cell death or cell vitality. With a concentration of Diflunisal corresponding to three bound molecules per albumin no substantial blockade of the respiration chain is registered. The deviation from the control (which means pH dependence of the respiration chain without distortion) is negligible. However, if the concentration of Diflunisal is changed to a ratio of 4:1 of Diflunisal to albumin, a significant increase in activity is found. When the Diflunisal concentration is further increased to a ratio of 6:1, the respiration blockade reaches an optimum up to pH 6.9. From pH 6.9 to 7.2 the blockade effect declines to ineffectiveness. If the concentration of Diflunisal is further increased corresponding to 7 to 9 Diflunisal to albumin, a new respiration blockade effect becomes apparent between pH 7.0-7.4, which corresponds to the apoptosis effect between pH 7.0-7.4 of figure 1.

This has been shown in the samples of the present invention as is referred to in figure 1 and figure 4.

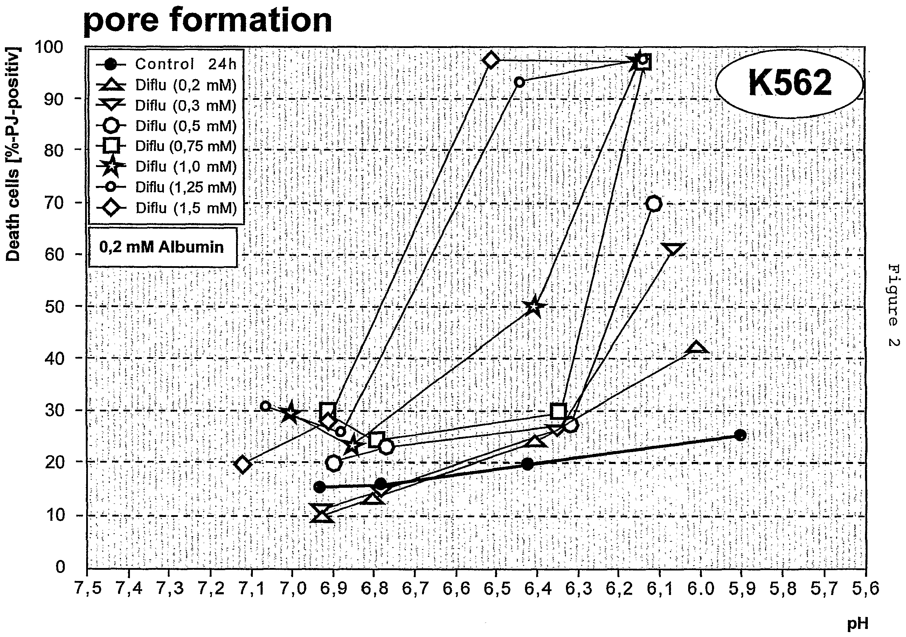

Additionally, experiments have been performed regarding the pore formation quality of Diflunisal. Pore formation can only directly be demonstrated with cancer cell suspensions by flow cytometer measurements as diagrammed in figure 2. Pore formation can be produced at pH < 7.0 and starts as the respiration chain inhibition at about 0.7 mM interstitial Diflunisal concentration.

The present invention thus provides an effective, highly selective pH-dependent cancer therapy which can be outlined as follows. A significant advantage of the present invention is that it is not restricted to the treatment of certain tumors, but that it is applicable to all kinds of tumors and metastases.

It should be pointed out that there exists a decisive difference to the application of Diflunisal and other NSAID-compounds as referred to in the introduction. In patient treatments, where 1000 mg/day of the compounds are administered, corresponding to < 0.3 mM interstitial concentrations (identical to Diflunisal to albumin ratio of < 2:1), no cancer cell kill takes place but only disturbance of the COX-systems, which leads to proliferation inhibition, i.e. to retardation of tumor growth.

In contrast to these procedures our invention provides real therapy effects by killing cancer cells selectively and pH-dependent. A further significant advantage of the present invention is that it is not restricted to the treatment of certain tumors but that it is applicable to all kinds of tumors and metastases.

Diflunisal should be administered to a patient in an amount so that in the interstitial milieu the molecular ratio of Diflunisal to albumin is from 4:1 to 6:1. (It is assumed that there will be a statistical distribution of the number of Diflunisal molecules which are bound to the interstitial albumin, but this does not affect the activity of the Diflunisal). Thus, a medicament should be adapted to provide this ratio of Diflunisal to albumin.

The usual average interstitial albumin concentration is 0.17 mM, which may vary slightly from organ to organ. It follows that the interstitial Diflunisal concentration should not exceed 1.1 mM and preferably should not be higher than 1.0 mM. The interstitial Diflunisal concentration should, however, not be allowed to fall below 0.55 mM, preferably 0.7 mM, more preferably 0.8 mM or 0.9 mM. Thus, according to the present invention Diflunisal should be administered to a patient in an amount so that the interstitial concentration of Diflunisal is from 0.55 mM to 1.1 mM, preferably from 0.7 mM to 1.1 mM, more preferably from 0.7 mM to 1.0 mM, e.g. 0.8 mM or 1.0 mM. The present invention also provides medicaments which are adapted to provide the above interstitial concentration of Diflunisal.

An interstitial concentration of Diflunisal of 1.1 mM corresponds to an oral dose (based on the bodyweight of the patient) of about 55 mg/kg, an interstitial concentration of Diflunisal of 1.0 mM corresponds to an oral dose of about 50 mg/kg, an interstitial concentration of Diflunisal of 0.55 mM corresponds to an oral dose of about 27,5 mg/kg, an interstitial concentration of Diflunisal of 0.7 mM corresponds to an oral dose of 35 mg/kg, an interstitial concentration of Diflunisal of 0.8 mM corresponds to an oral dose of about 40 mg/kg and an interstitial concentration of 0.9 mM corresponds to an oral dose of about 45 mg/kg. The above values are based on a female patient with 65 ml blood per kg, and in case of male patients with e.g. 77 ml blood per kg the dose has to be increased by about 18%. A skilled person can easily adapt the dose which is best suited to treat an individual patient based on the above information.

During the treatment period the interstitial concentration of Diflunisal should not exceed a molecular ratio to interstitial albumin of 6:1. Therefore, Diflunisal should preferably be administered only in such amounts which are necessary to replace Diflunisal which has left the interstitial milieu e.g. by metabolisation or by excretion and by action.

The administration of the Diflunisal should assure that the desired interstitial concentration of Diflunisal is maintained during the treatment, and the medicament should be adapted accordingly. In a suitable application scheme an initial high dose Diflunisal is administered, followed by lower doses which should maintain a steady-state level of interstitial Diflunisal. A suitable initial dose is preferably 30 mg/kg p.o to 40 mg/kg p.o, e.g. about 35 mg/kg p.o and after 12 hours another dose of preferably 25 mg/kg p.o to 35 mg/kg p.o, e.g. about 30 mg/kg p.o can be given and again every 12 hours 20 mg/kg p.o to 30 mg/kg p.o, e.g. about 25 mg/kg p.o, can be given. Preferred is the following dosage scheme:
a) administering to a patient an initial dose of 25 to 45 mg/kg bodyweight,
b) administering to the patient about 12 hours after the initial dose a second dose of Diflunisal of 20 mg/kg to 40 mg/kg bodyweight,
c) administering to the patient about 3 hours after the second dose a third dose of Diflunisal of 4 mg/kg to 8 mg/kg bodyweight,
d) administering to the patient about 3 hours after the third dose a fourth dose of Diflunisal of 4 mg/kg to 8 mg/kg bodyweight,
e) administering to the patient about 3 hours after the fourth dose a fifth dose of Diflunisal of 4 mg/kg to 8 mg/kg bodyweight,
f) administering to the patient about 3 hours after the fifth dose a sixth dose of Diflunisal of 4 mg/kg to 8 mg/kg bodyweight,
g) repeating the cycle 12 hours after the sixth dose starting at step b) above.

A variation of this scheme can be: the dosage of step d) could be 8 to 10 mg/kg and the dosage of step f) as a consequence be cancelled.

The most preferred dosage scheme is as follows (for a female patient with 65 ml blood per kg body weight, for other patients the dosage scheme can be adapted, if necessary):

| Time | Dose | Variation |
|---|---|---|
| initial dose | about 35 mg/kg | |
| after about 12 hours | about 30 mg/kg | |
| after about 3 hours | about 6 mg/kg | |
| after about 3 hours | about 6 mg/kg | 12 mg/kg |
| after about 3 hours | about 6 mg/kg | |
| after about 3 hours | about 6 mg/kg | 0 mg/kg |
| after about 12 hours | about 30 mg/kg | |
| after about 3 hours | about 6 mg/kg | |
| after about 3 hours | about 6 mg/kg | 12 mg/kg |
| after about 3 hours etc. | about 6 mg/kg | |

Of course, other dosage schemes are also possible, if it is ensured that the interstitial Diflunisal concentration is as disclosed above. With the information provided in this specification, a skilled person can easily find amended dosage schemes which provide the required interstitial concentration of Diflunisal. Instead of the above mentioned 3 hour intervals four hour, five hour or six hour intervals can be used or 12 hour intervals can be used with adapting the dosage which is given after each interval. The dosage for each interval can also be adapted depending on the patient and in particular on the amount of blood per kg bodyweight of this patient as explained above, and such variations and adaptations are within the knowledge of a skilled person.

The kind of oral medicament which can be used according to the invention is not particularly restricted, as long as it is adapted to be applied according to the dosage scheme as explained above.

A preferred route of administration of the Diflunisal is orally as explained above. A further preferred route of administration is parenterally, in particular intravenously. As with the oral application, the preferred parenteral administration is in intervals. Suitable doses of Diflunisal are such to ensure the above mentioned interstitial Diflunisal concentration and can be determined by a skilled person on the basis of the information provided in the present specification and his general technical knowledge.

A suitable initial dose by parenteral, in particular intravenous, administration is from 30 mg/kg i.v. to 40 mg/kg i.v., e.g. about 35 mg/kg i.v., followed after about 12 hours by a dose from 25 mg/kg i.v. to 35 mg/kg i.v., e.g. about 30 mg/kg i.v. followed after about 12 hours by a dose from 25 mg/kg i.v. to 35 mg/kg i.v., e.g. about 30 mg/kg i.v. This dose is administered about every 12 hours.

It is also preferred according to the present invention that the Diflunisal is administered in a combination of oral administration and parenteral application.

A suitable combined oral and parenteral dosage scheme is an initial dose from 30 to 40 mg/kg p.o, such as 35 mg/kg p.o, after about 12 hours from 25 to 35 mg/kg p.o, e.g. about 30 mg/kg p.o and in addition from 20 to 30 mg/kg i.v., e.g. about 25 mg/kg i.v. and then about every 24 hours a dose from 25 to 35 mg/kg p.o, e.g. about 30 mg/kg p.o and in addition a dose from 20 to 30 mg/kg i.v., such as about 25 mg/kg i.v.

Other suitable dosage schemes can easily be found by a skilled person based on the above information and bearing in mind that according to the invention it is important to maintain an interstitial concentration of Diflunisal to albumin from 4:1 to 6:1 on a molecular ratio.

The compositions used in the invention can be formulated in a known manner for medicaments for mammals, preferably man. In the medicament the compositions are present as a mixture with one or more pharmaceutically acceptable organic or inorganic excipients which are suitable for enteral or parenteral administration. The oral administration of the compositions by means of tablets, capsules, powders or in liquid form such as suspensions, in solution or as an emulsion or as syrup is particularly preferred.

In the case of formulation as tablets, customary pharmaceutical excipients such as sodium citrate, lactose, microcrystalline cellulose and starch, lubricants such as unhydrated silicic acid, hydrogenated castor oil, magnesium stearate, sodium lauryl sulfate and talcum as well as binders such as starch, glucose, lactose, gum arabic, mannitol, magnesium trisilicate and the like are used. If the compositions according to the invention are to administered by means of liquids, customary excipients for liquid formulations can be used.

A formulation for injection and infusions is likewise preferred and can be prepared as is known and described in the relevant standard textbooks.

It is also possible and preferred to prepare the medicaments for use in the invention in the form of depot formulations which release the active ingredient, i.e. the Diflunisal according to the above dosage scheme, and the preparation of such depot formulations or sustained release preparations or delayed-release preparations, is well known to a skilled person.

By this way of therapy when proceeded over several days, the lactate transporting cancer cells are killed gradually and the tumor milieu will become more and more alkaline. Those cancer cells who survive will then respirate in an alkaline milieu. In this connection it can also be referred to Muller, B., Fischer, B., Kreutz, W.; Immunology 2000, 99, 375-364, where it is shown that only cancer cells which proliferate in an alkaline milieu can be attacked by the immune system and that those cancer cells which proliferate in acidic milieu survive. This means if the therapy proposed is continued over many days, at least after more than six days of immune-induction time, the natural immune system should start to kill the alkaline tumor fraction. Proceeding the therapy, in the end the tumor should go to total remission.

The invention is further explained in the following experiments which are, however, not intended to limit the scope of protection.

The results of the experiments are shown in the figures.
- Figure 1:: Figure 1 is a representation of apoptosis measurements with a bladder cancer cell line RT112. It is plotted the extent of produced DNA fragments (nucleosomes and oligonucleosomes) of killed cancer cells in dependence of pH and Diflunisal concentration. The albumin concentration in all measurements was kept at 0.06 mM. Besides the control measurements without Diflunisal, there is also a "positive" control (CAM) registered with a defined calibrated system.
- Figure 2:: Figure 2 is a representation of pore formation under conditions of interstitial albumin concentration of 0.2 mM. It is plotted the percentage of PJ-dyed dead cells in dependence of pH and Diflunisal concentration. The dead cell fraction compared to the total cell number is measured in a flow cytometer. The experiments were conducted with the leukemia cell line K-562. Of special interest is the pore formation starting with a pH characteristic which corresponds to the third affinity level of albumin at pH ≤ 7.0, when 1.25 mM to 1.5 mM Diflunisal is applied. Of minor interest is the second pore formation process starting at pH 6.4, because this process functions beyond the acidity milieu of most tumors. At a concentration of 1.0 mM Diflunisal there is a transition situation where both transport systems are superimposed. Not shown is a third transport system starting at a pH of 7.2-7.3 corresponding to the transmission from the fourth affinity level of albumin.
- Figure 3:: Figure 3 shows the measurement of the lactate transport with the bladder cancer cell line RT112. There is plotted a control measurement which indicates lactate efflux in dependence of pH and without any Diflunisal influence. By the action of Diflunisal the lactate efflux is remarkably enhanced from pH 6.0 to 7.2, indicating Diflunisal influx (antiport) into the cancer cells and leading as a consequence to apoptosis within this pH range.
- Figure 4:: Figure 4 shows the indirect registration of the apoptosis activity by measurement of the activity of the mitochondrial respiration chain. With interstitial albumin conditions (0.2 mM) it is demonstrated that with 1.0 mM Diflunisal the deviation from normal respiration activity (control) starts at about pH 7.2 and reaches its maximum at about pH 6.9, which means that the inhibition of the respiration activity starts at about pH 7.2 and is cancelled totally at pH ≤ 6.9. This behavior reflects the apoptosis induction from the second affinity level of albumin. Increasing the concentrations higher than 1.0 mM also the whole respiration activity within the pH range 7.0-7.4 is diminished and falls to 0 at 1.5 mM. The same is true with concentrations up to 2.0 mM. This effect is introduced by apoptosis at pH 7.0-7.4, accelerated by the third and fourth affinity level of albumin. The control measurements represent the respiration chain activity in dependence of pH without Diflunisal distortion.

### Examples

### General

The human bladder tumor cell line RT112 or the human chronic myeloid leukemia cell line K562 were purchased from DKFZ Tumorzell- und datenbank (Institut für experimentelle Pathologie, Heidelberg, Germany.) or DSM (Deutsche Sammlung von Mikroorganismen und Zellkulturen GmbH, Braunschweig, Germany), supplemented with 2 mM L-glutamine (available from the company BIOCHROM, Berlin, Germany), 1% NEAE (BIOCHROM), 10% heat-inactivated fetal calf serum (BIOCHROM), 100 U/ml penicillin and 50 µg/ml streptomycin (BIOCHROM) at 37°C and 5% CO₂. Only mycoplasma-free cell cultures were used. This was frequently tested using a specific detection kit provided by BOEHRINGER MANNHEIM, Mannheim, Germany.

### Apoptosis

Apoptosis was measured by determination of cytoplasmic histone-associated DNA fragments (mono- and oligonucleosomes) after induced cell death. The qualitative and quantitative in vitro determination of apoptosis in RT112 tumor cells was analyzed with a photometric enzyme immunoassay. In brief, RT112 cells were harvested, washed twice and resuspended in culture media to yield a density of 2.5 x 10⁵/ml. 100 µl/well of the RT112 cell suspension were seeded into 96 well microtiter plates (obtainable from BECTON DICKINSON) and incubated overnight at 37°C and 5% CO₂. The supernatant was replaced by 90 µl pH-adjusted culture medium alone (control). Again, the cells were incubated for various time periods at 37°C and 5% CO₂. Supernatants of the cell cultures were discarded and RT112 cells were lysed in lysis buffer for 30 minutes at RT (25°C). The cytoplasmic fractions were transferred into a streptavidin-coated microtiter plate, and the working procedure for the ELISA was performed according to the manufacturer's instructions (the ELISA test was obtained from BOEHRINGER MANNHEIM, Germany). The results of the tests are shown in figure 1.

It can be seen that Diflunisal induces pH-dependent apoptosis in lactate transporting cells. Applying 0.3 mM Diflunisal and 0.06 mM albumin on bladder cancer cells (RT112) apoptosis is dominantly caused by the second affinity level of albumin. But there is also a superimposed part of the third level. Using 0.5 mM Diflunisal apoptosis is dominantly exhibited from the third affinity level and finally at 1.0 mM apoptosis is exclusively determined by the third affinity level, starting at pH 7.0 and ending up at pH 7.4.

### Pore formation

Pore formation was measured by determination of the necrotic cells measured by flow cytometry. Cultures of the leukemia cell line K-562 were harvested, and 5 x 10⁵ cells were added into 6 ml tubes (BD). After centrifugation (250 x g; 5 min; room temperature (25°C)) the cells were resuspended in 900 µl pH-adjusted culture medium (pH 6.0-7.4; RPMI 1640 without NaHCO₃; available from SIGMA). Then test substances were added to the samples and cells were incubated in a final volume of 1 ml at 37°C in a water bath. After the incubation period the pH of the samples was measured, and the cells were washed and resuspended in 300 µl FACS medium (PBS + 2% FCS and 0.1% NaN₃). Cells were stained with propidium iodide (PI; available form SIGMA; 25 µl/25 µg/tube) for 15 minutes at RT (25°C). Subsequently analysis of the stained cells was performed on an EPICS XL-MCL flow cytometer (available from COULTER, Krefeld, Germany), equipped with a 15 mW argon laser at an excitation wavelength of 488 nm. PI fluorescence was measured in the red channel (FL3; 620 nm longpass filter). Data acquisition and analysis was performed using XL-System II software (available from the company COULTER). The results are shown in figure 2.

It can be seen that Diflunisal causes pH-dependent pore formation in cancer cells. Figure 2 shows that pore formation starts in a therapy-relevant pH range from an interstitial concentration starting at 1.25 mM onwards.

### Lactate transport

The lactate transport was measured by qualitative enzymatic determination of lactate in supernatants of RT112 cells at 340 nm. RT112 cells were harvested, washed twice and resuspended in culture media to yield a density of 2 x 10⁵/ml. 500 µl/well of the RT112 cell suspension were seeded into 24 well microtiter plates (BD) and incubated overnight at 37°C and 5% CO₂. The supernatant was replaced by 450 µl pH-adjusted culture medium (pH 6.0-7.4) and 50 µl of the solution of preferred substances or 500 µl/well pH-adjusted culture medium alone (control). Again, the cells were incubated for 24 h at 37°C and 5% CO₂. The measurement of lactate in the supernatants at 340 nm (PERKIN-ELMER, Lambda 17. UV-VIS Spectrophotometer, Überlingen, Germany) was performed according to the manufacturer's instructions (SIGMA DIAGNOSTICS, Deisenhofen, Germany; Lactate: Quantitative, Enzymatic Determination of Lactate in Whole Blood at 340 nm (Procedure No. 826-UV)). The results of this experiment are shown in figure 3.

Figure 3 shows that Diflunisal influx into the cancer cells is mediated by albumin and is coupled to lactate efflux, which means that Diflunisal influx is coupled as an antiport to lactate efflux. It can be seen that with three Diflunisal bound to albumin occupying the first affinity stage no antiport happens, while with four to six bound Diflunisal, i.e. from the second affinity stage, effective apoptosis is performed from pH 6.0-7.2.

### Respiration

The mitochondrial activity of RT112 was determined using a colorimetric assay (XTT assay).

The non-radioactive, colorimetric assay system using XTT (sodium 3-[1-(phenylaminocarbonyl)-3,4-tetrazolium]-bis (4-methoxy-6-nitro) benzene sulfonic acid hydrate) (BOEHRINGER MANNHEIM) was used to evaluate the influence of cytotoxic molecules on the mitochondrial activity of human tumor cells. The mitochondrial succinate-tetrazolium reductase system cleaves the tetrazolium salt XTT to formazan. The amount of this dye is directly correlated to the number of metabolically active cells, as the enzyme system is only active in respirating and viable cells. For this purpose, RT112 cells were harvested, washed twice and resuspended in culture media to yield a density of 2.5 x 10⁵/ml. 100 µl/well of the RT112 cell suspension were seeded into 96 well microtiter plates (BECTON DICKINSON (BD), Heidelberg, Germany) and incubated overnight at 37°C and 5% CO₂. The supernatant was replaced by 90 µl pH-adjusted culture medium (pH 6.0-7.4) and 10 µl of preferred substances or 100 µl/well pH-adjusted culture medium alone (control). The cells were incubated for various time periods at 37°C and 5% CO₂. Thereafter, cells were washed twice and reincubated in fresh pH-adjusted culture medium. After the incubation period, pH of each well was determined with highly sensitive microelectrodes before 50 µl XTT labeling mixture was added. The cells were further incubated for additional 4 h at 37°C and 5% CO₂. The spectrophotometric absorbance of the samples was measured using a microtiter plate (ELISA) reader (DYNATECH, Denkendorf, Germany) at a wavelength of 490 nm (630 nm as reference). Data acquisition and analysis was performed using BioLinx 2.1 software (DYNATECH). The results of this experiment are shown in figure 4.

## Claims

1. Diflunisal for use in the treatment of cancer, wherein the Diflunisal-containing medicament is adapted for administration so that a molecular ratio of 4:1 to 6:1 of interstitial Diflunisal:interstitial albumin is provided, wherein the interstitial albumin concentration is measured in the lymph fluid system.

2. Diflunisal for use in the treatment of cancer, wherein the Diflunisal-containing medicament is adapted for the administration so that an interstitial Diflunisal concentration of 0.55 to 1.1 mM is provided.

3. Diflunisal for use according to claim 2, wherein the Diflunisal-containing medicament is adapted for administration so that an interstitial Diflunisal concentration of 0.8 to 1.0 mM is provided.

4. Diflunisal for use according to any of claims 1 to 3, wherein the Diflunisal-containing medicament is adapted to provide a dosage scheme which constantly maintains a molecular ratio of 4:1 to 6:1 of interstitial Diflunisal:interstitial albumin.

5. Diflunisal for use according to any of claims 1 to 4, wherein the Diflunisal-containing medicament is adapted to provide an initial high dose of Diflunisal and following lower doses of Diflunisal to maintain a constant interstitial Diflunisal concentration.

6. Diflunisal for use according to any of claims 1 to 5, wherein the Diflunisal-containing medicament is for oral application and is adapted to provide the following dosage regime comprising
a) administering to a patient an initial dose of 25 to 45 mg/kg bodyweight p.o,
b) administering to the patient about 12 hours after the initial dose a second dose of Diflunisal of 20 mg/kg to 40 mg/kg bodyweight p.o,
c) administering to the patient about 3 hours after the second dose a third dose of Diflunisal of 4 mg/kg to 8 mg/kg bodyweight p.o,
d) administering to the patient about 3 hours after the third dose a fourth dose of Diflunisal of 4 mg/kg to 8 mg/kg bodyweight p.o,
e) administering to the patient about 3 hours after the fourth dose a fifth dose of Diflunisal of 4 mg/kg to 8 mg/kg bodyweight p.o,
f) administering to the patient about 3 hours after the fifth dose a sixth dose of Diflunisal of 4 mg/kg to 8 mg/kg bodyweight p.o,
g) repeating the cycle after 12 hours of the sixth dose starting at step b) above.

7. Diflunisal for use according to any of claims 1 to 5, wherein the Diflunisal-containing medicament is for parenteral administration and is adapted to provide the following dosage regime comprising:
a) administering to a patient an initial dose of Diflunisal of 30 to 40 mg/kg bodyweight i.v.,
b) administering to the patient about 12 hours after the initial dose a second dose of Diflunisal of 25 mg/kg to 35 mg/kg bodyweight i.v.,
c) administering to the patient about every 12 hours a further dose of Diflunisal of 25 mg/kg to 35 mg/kg bodyweight i.v.

8. Diflunisal for use according to any of claims 1 to 5, wherein the Diflunisal-containing medicament is adapted to be administered partially orally and partially parenterally and which provides the following dosage regime comprising:
a) administering to a patient an initial dose of Diflunisal of 30 to 40 mg/kg bodyweight p.o,
b) administering to the patient about 12 hours after the initial dose a second dose of Diflunisal of 25 mg/kg to 35 mg/kg bodyweight p.o and in addition 25 mg/kg to 35 mg/kg bodyweight i.v.,
c) administering to the patient about 24 hours after the second dose a third dose of Diflunisal of 25 mg/kg to 35 mg/kg bodyweight p.o and in addition 20 mg/kg to 30 mg/kg bodyweight i.v.,
d) administering to the patient about every 24 hours a further dose of Diflunisal of 25 mg/kg to 35 mg/kg bodyweight p.o and in addition 20 mg/kg to 30 mg/kg bodyweight i.v.

## Patentansprüche

1. Diflunisal zur Verwendung bei der Behandlung von Krebs, wobei das Diflunisal-haltige Medikament für die Verabreichung angepasst ist, so dass ein Molekularverhältnis von 4 : 1 bis 6 : 1 von interstitiellem Diflunisal : interstitiellem Albumin bereitgestellt wird, wobei die Konzentration an interstitiellem Albumin im Lymphflüssigkeitssystem gemessen wird.

2. Diflunisal zur Verwendung bei der Behandlung von Krebs, wobei das Diflunisal-haltige Medikament für die Verabreichung angepasst ist, so dass eine Konzentration an interstitiellem Diflunisal von 0,55 bis 1,1 mM bereitgestellt wird.

3. Diflunisal zur Verwendung nach Anspruch 2, wobei das Diflunisal-haltige Medikament für die Verabreichung angepasst ist, so dass eine Konzentration an interstitiellem Diflunisal von 0,8 bis 1,0 mM bereitgestellt wird.

4. Diflunisal zur Verwendung nach einem der Ansprüche 1 bis 3, wobei das Diflunisal-haltige Medikament so angepasst ist, dass ein Dosierschema bereitgestellt wird, das konstant ein Molekularverhältnis von 4 : 1 bis 6 : 1 an interstitiellem Diflunisal : interstitiellem Albumin aufrechterhält.

5. Diflunisal zur Verwendung nach einem der Ansprüche 1 bis 4, wobei das Diflunisal-haltige Medikament so angepasst ist, dass zunächst eine hohe Dosis an Diflunisal und anschließend geringere Dosen an Diflunisal zur Aufrechterhaltung einer konstanten Konzentration an interstitiellem Diflunisal bereitgestellt werden.

6. Diflunisal zur Verwendung nach einem der Ansprüche 1 bis 5, wobei das Diflunisal-haltige Medikament für die orale Anwendung gedacht und so angepasst ist, dass das folgende Dosierregime bereitgestellt wird, umfassend
a) das Verabreichen einer Anfangsdosis von 25 bis 45 mg/kg Körpergewicht p.o. an einen Patienten,
b) das Verabreichen einer zweiten Dosis Diflunisal von 20 mg/kg bis 40 mg/kg Körpergewicht p.o. an den Patienten etwa 12 Stunden nach der Anfangsdosis,
c) das Verabreichen einer dritten Dosis Diflunisal von 4 mg/kg bis 8 mg/kg Körpergewicht p.o. an den Patienten etwa 3 Stunden nach der zweiten Dosis,
d) das Verabreichen einer vierten Dosis Diflunisal von 4 mg/kg bis 8 mg/kg Körpergewicht p.o. an den Patienten etwa 3 Stunden nach der dritten Dosis,
e) das Verabreichen einer fünften Dosis Diflunisal von 4 mg/kg bis 8 mg/kg Körpergewicht p.o. an den Patienten etwa 3 Stunden nach der vierten Dosis,
f) das Verabreichen einer sechsten Dosis Diflunisal von 4 mg/kg bis 8 mg/kg Körpergewicht p.o. an den Patienten etwa 3 Stunden nach der fünften Dosis,
g) das Wiederholen des Zyklus 12 Stunden nach der sechsten Dosis, beginnend bei dem obigen Schritt b).

7. Diflunisal zur Verwendung nach einem der Ansprüche 1 bis 5, wobei das Diflunisal-haltige Medikament für die parenterale Verabreichung gedacht und so angepasst ist, dass das folgende Dosierregime bereitgestellt wird, umfassend:
a) das Verabreichen einer Anfangsdosis Diflunisal von 30 bis 40 mg/kg Körpergewicht i.v. an einen Patienten,
b) das Verabreichen einer zweiten Dosis Diflunisal von 25 mg/kg bis 35 mg/kg Körpergewicht i.v. an den Patienten etwa 12 Stunden nach der Anfangsdosis,
c) das Verabreichen einer weiteren Dosis Diflunisal von 25 mg/kg bis 35 mg/kg Körpergewicht i. v. an den Patienten etwa alle 12 Stunden.

8. Diflunisal zur Verwendung nach einem der Ansprüche 1 bis 5, wobei das Diflunisal-haltige Medikament für die teilweise orale und teilweise parenterale Verabreichung angepasst ist und das folgende Dosierregime vorsieht, umfassend:
a) das Verabreichen einer Anfangsdosis Diflunisal von 30 bis 40 mg/kg Körpergewicht p.o. an einen Patienten,
b) das Verabreichen einer zweiten Dosis Diflunisal von 25 mg/kg bis 35 mg/kg Körpergewicht p.o. und außerdem 25 mg/kg bis 35 mg/kg Körpergewicht i.v. an den Patienten etwa 12 Stunden nach der Anfangsdosis,
c) das Verabreichen einer dritten Dosis Diflunisal von 25 mg/kg bis 35 mg/kg Körpergewicht p.o. und außerdem 20 mg/kg bis 30 mg/kg Körpergewicht i.v. an den Patienten etwa 24 Stunden nach der zweiten Dosis,
d) das Verabreichen einer weiteren Dosis Diflunisal von 25 mg/kg bis 35 mg/kg Körpergewicht p.o. und außerdem 20 mg/kg bis 30 mg/kg Körpergewicht i.v. an den Patienten etwa alle 24 Stunden.

## Revendications

1. Diflunisal pour utilisation dans le traitement du cancer, dans lequel le médicament contenant du Diflunisal est adapté à l'administration de sorte que soit obtenu un rapport moléculaire de 4:1 à 6:1 du Diflunisal interstitiel à l'albumine interstitielle, dans lequel la concentration d'albumine interstitielle est mesurée dans le système lymphatique.

2. Diflunisal pour utilisation dans le traitement du cancer, dans lequel le médicament contenant du Diflunisal est adapté à l'administration de sorte que soit obtenue une concentration de Diflunisal interstitiel de 0,55 à 1,1 mM.

3. Diflunisal pour utilisation selon la revendication 2, dans lequel le médicament contenant du Diflunisal est adapté pour l'administration de sorte que soit obtenue une concentration de Diflunisal interstitiel de 0,8 à 1,0 mM.

4. Diflunisal pour utilisation selon l'une quelconque des revendications 1 à 3, dans lequel le médicament contenant du Diflunisal est adapté pour fournir un schéma de dosage qui maintient constamment un rapport moléculaire de 4:1 à 6:1 de Diflunisal interstitiel à l'albumine interstitielle.

5. Diflunisal pour utilisation selon l'une quelconque des revendications 1 à 4, dans lequel le médicament contenant du Diflunisal est adapté pour fournir une dose élevée initiale de Diflunisal et ensuite des doses de Diflunisal inférieures pour maintenir une concentration constante de Diflunisal interstitiel.

6. Diflunisal pour utilisation selon l'une quelconque des revendications 1 à 5, dans lequel le médicament contenant du Diflunisal est destiné à une application orale et est adapté pour fournir le régime de dosage suivant comprenant :
a) l'administration à un patient d'une dose initiale de 25 à 45 mg/kg de poids corporel par voie orale,
b) l'administration au patient environ 12 heures après la dose initiale d'une deuxième dose de Diflunisal de 20 mg/kg à 40 mg/kg de poids corporel par voie orale,
c) l'administration au patient environ 3 heures après la deuxième dose d'une troisième dose de Diflunisal de 4 mg/kg à 8 mg/kg de poids corporel par voie orale,
d) l'administration au patient environ 3 heures après la troisième dose d'une quatrième dose de Diflunisal de 4 mg/kg à 8 mg/kg de poids corporel par voie orale,
e) l'administration au patient environ 3 heures après la quatrième dose d'une cinquième dose de Diflunisal de 4 mg/kg à 8 mg/kg de poids corporel par voie orale,
f) l'administration au patient environ 3 heures après la cinquième dose d'une sixième dose de Diflunisal de 4 mg/kg à 8 mg/kg de poids corporel par voie orale,
g) la répétition du cycle après 12 heures de la sixième dose en partant de l'étape b) ci-dessus.

7. Diflunisal pour utilisation selon l'une quelconque des revendications 1 à 5, dans lequel le médicament contenant du Diflunisal est adapté pour l'administration parentérale et est adapté pour le régime de dosage suivant comprenant :
a) l'administration à un patient d'une dose initiale de Diflunisal de 30 à 40 mg/kg de poids corporel par voie intraveineuse,
b) l'administration du patient environ 12 heures après la dose initiale d'une deuxième dose de diflunisal de 25 mg/kg à 35 mg/kg de poids corporel par voie intraveineuse,
c) l'administration au patient environ toutes les 12 heures d'une autre dose de diflunisal de 25 mg/kg à 35 mg/kg de poids corporel par voie intraveineuse.

8. Diflunisal pour utilisation selon l'une quelconque des revendications 1 à 5, dans lequel le médicament contenant du Diflunisal est adapté pour être administré en partie par voie orale et en partie par voie parentérale et qui fournit le régime de dosage suivant comprenant :
a) l'administration à un patient d'une dose initiale de Diflunisal de 30 à 40 mg/kg de poids corporel par voie orale,
b) l'administration au patient environ 12 heures après la dose initiale d'une deuxième dose de Diflunisal de 25 mg/kg à 35 mg/kg de poids corporel par voie orale et en plus de 25 mg/kg à 35 mg/kg de poids corporel par voie intraveineuse,
c) l'administration au patient environ 24 heures après la deuxième dose d'une troisième dose de Diflunisal de 25 mg/kg à 35 mg/kg de poids corporel par voie orale et en plus de 20 mg/kg à 30 mg/kg de poids corporel par voie intraveineuse,
d) l'administration au patient environ toutes les 24 heures d'une autre dose de Diflunisal de 25 mg/kg à 35 mg/kg de poids corporel par voie orale et en plus de 20 mg/kg à 30 mg/kg de poids corporel par voie intraveineuse.
